(19) Europäisches Patentamt
European Patent Office
Office européen des brevets

(11) Publication number: 0 124 231 B1

(12) **EUROPEAN PATENT SPECIFICATION**

(45) Date of publication of patent specification: 22.07.87

(51) Int. Cl.[4]: C 07 D 301/19, C 07 D 303/04, B 01 J 31/18

(21) Application number: 84301864.9

(22) Date of filing: 20.03.84

(54) **Epoxidation of ethylene.**

(30) Priority: 23.03.83 GB 8307976

(43) Date of publication of application: 07.11.84 Bulletin 84/45

(45) Publication of the grant of the patent: 22.07.87 Bulletin 87/30

(84) Designated Contracting States: BE DE FR GB IT NL

(56) References cited:
BE-A- 735 842
DE-A-2 207 506
US-A-3 489 775
US-A-4 157 346

(73) Proprietor: BP Chemicals Limited
Belgrave House 76 Buckingham Palace Road
London, SW1W 0SU (GB)

(72) Inventor: Kelly, Raymond Leslie
BP Chemicals Limited Saltend
Hedon Hull HU12 8DS (GB)

(74) Representative: Crack, Richard David et al
c/o The British Petroleum Company plc
Patents Division Chertsey Road
Sunbury-on-Thames Middlesex, TW16 7LN (GB)

EP 0 124 231 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process of epoxidising ethylene using hydroperoxides as the epoxidising agents.

Epoxidation of unsaturated compounds, especially olefins, gives rise to epoxides which are the basic monomers for industries based on epoxide polymers and copolymers e.g. epoxy resins, surfactants, adhesives and elastomeric compositions. Ethylene oxide, propylene oxide and cyclohexene oxide are but a few specific examples of such epoxide monomers.

In liquid phase epoxidation processes, the choice of catalyst must depend upon the following factors:

(a) its inertness towards the liquid reaction medium,

(b) the speed of reaction, the rate of the selectivity to the desired epoxide product,

(c) the cost of the catalyst,

(d) the ease of its recovery from the reaction mixture, and

(e) its stability in the presence of the hydroperoxide epoxidising agents.

Ideally it would be desirable to convert all the hydroperoxide used into the epoxide in the shortest possible time with maximum selectivity to the olefin oxide. For simple olefins the order of reactivity is related to the number of alkyl substituents coordinated to the carbon-carbon double bond. Ethylene has no such substituents and is a particularly difficult olefin to epoxidise. Of the several catalysts that have been proposed for the epoxidation of olefins, compounds of the metals in Group V and VI of the Periodic Table have been most effective. Specifically, compounds of molybdenum, vanadium and tungsten have been found to be most suitable. Catalyst compositions containing molybdenum whether in the form of inorganic compounds or as organo-metallic complexes have given the best results. Of these, complexes of molybdenum oxide with organic nitrogen containing bases have been the most successful. British Patent Specification No. 1365589 describes the use of complexes of this type in such a reaction. In general, however, these catalysts show poor conversion and selectivity to the epoxide when the olefin used is ethylene.

The object of the present invention, therefore, is to provide complexes for use as catalysts for the epoxidation of ethylene which enable improved conversion and selectivity to ethylene oxide to be achieved.

Accordingly, the present invention comprises a process for the epoxidation of ethylene in the liquid phase using a hydroperoxide as an epoxidising agent and, as catalyst, an effective amount of a chelate complex of a Group VB or VIB metal and a bidentate nitrogenous ligand characterised in that the bidentate nitrogenous ligand has the general formula:

$$HO—Ar—CH{=}NR$$

wherein Ar is an arylene radical with the OH group and the —CH=NR group in an ortho relationship,

R is selected from the groups consisting of OH, —Ar—X and $—R_1—N{=}CH—Ar—OH$ in which

—Ar has the same significance as before,

X is an —OH or a —COOH group and

$R_1$ is either an arylene group or a $—(CH_2)_n—$ group in which n is an integer selected from 0, 1, 2, 3 and 4.

Examples of nitrogenous bidentate ligands include HO—Ar—CH=N—OH (Salicylaldoxime), HO—Ar—CH=N$^-$Ar—OH (N-salicylidene-2-aminophenol, hereafter referred to as SAP), HO—Ar—CH=N—Ar—COOH (N-salicylidene-2-aminobenzoic acid, hereafter referred to as SAN), HO—Ar—CH=N—Ar—N=CH—Ar—OH (phenylene bis(salicylimine), hereafter referred to as SALOPH), $HO—Ar—CH{=}N—(CH_2)_2—N{=}CH—Ar—OH$ (ethylene bis(salicylimine), hereafter referred to as SALEN).

In each of these compounds the substituents on the aryl nuclei are preferably in the ortho-position. These bases may be complexed either as such or in combination with other conventional ligands such as acetylacetonates.

The Group VB and VIB metals referred to herein are suitably used as their oxides. The metals are suitably selected from molybdenum, vanadium and tungsten, and the preferred metal is molybdenum.

The chelate complexes are used in the liquid phase with hydroperoxides in the conventional epoxidation reactions.

Any of the conventional hydroperoxides may be used. It is preferable to use tert-butyl hydroperoxide.

Examples of solvents that may be used as the reaction medium for the liquid phase epoxidation are hydrocarbons such as benzene, cyclohexane and cyclohexene; halogenated hydrocarbons such as dichlorobenzene; and alcohols such as tertiary butanol.

It is preferable however to use a solvent which is stable towards oxidation under the reaction conditions.

Thus, the ethylene, the chelate complex and the hydroperoxide may be dissolved in an oxidant-stable organic solvent and placed in a reactor equipped with a stirrer and thermometer. The molar ratio of the chelate complex to the hydroperoxide is suitably between 0.005 and 0.1, preferably between 0.02 and 0.05, most preferably 0.01.

An excess of the solvent may be used in relation to the hydroperoxide. For instance, between 1.2 and 50 volumes of the solvent may be used per volume of the hydroperoxide.

The epoxidation reaction occurs in the presence of an excess of ethylene in relation to the hydroperoxide so as to increase the utilisation of the latter. The ethylene to hydroperoxide molar ratio is suitably between 0.5 and 100, preferably between 2 and 20.

The reaction mixture is suitably heated to a temperature of above 50°C, preferably between 50 and 150°C for the duration of the reaction to achieve high conversion of the hydroperoxide.

The reaction may be carried out at atmospheric pressure or, as in the case where one of the reactants is a gas, at elevated pressure up to 100 bar.

The sequence in which the reactants are added is not significant. However, it is preferable to mix the chelate complex and the hydroperoxide first before adding either component to the ethylene.

A further surprising feature of the present invention is the improved ability of the catalyst to epoxidise the relatively inert olefin ethylene in the preence of an agent such as cyclohexene oxide. Thus if the mixture of the chelate complex and the hydroperoxide is added to a mixture of ethylene with cyclohexene oxide, the conversion and selectivity to ethylene oxide is further improved. The product epoxide, i.e. ethylene oxide may be used as an autocatalytic agent instead of cyclohexene oxide. The amount of epoxide added to the reaction mixture will depend upon the olefin being epoxidised. In general it is suitably between 1 and 10 moles per mole of the catalyst.

The reaction may be carried out either batchwise or continuously.

The present invention is further illustrated with reference to the following Examples.

Major differences in both activity and selectivity have been observed in the catalytic activity of molybdenum catalysts depending on the actual nitorgenous chelating ligand coordinated. Examples 1—3 [(a) without added epoxide, (b) with added epoxide] illustrate catalysts which are selective to ethylene oxide particularly when an epoxide is added with the catalyst. Less selective are the catalysts of Examples 4 and 5 [(a) without added epoxide, (b) with added epoxide]. However, as Examples 6 and 7 show those complexes which use nitrogenous bases outside those described herein are much less selective towards ethylene oxide.

In the Examples, some of the ligands are abbreviated as follows:

N-Salicylidene-2-aminophenol as "SAP"
N-Salicylidene-2-aminobenzoic acid as "SAN"
Phenylene bis(salicylimine) as "SALOPH"
Ethylene bis(salicylimine) as "SALEN"
4-phenyliminopentanone-2 as "PIP"
3-phenylimino-1-phenylbutanone-1 as "PPB"
Acetylacetonate as "acac".

Thus, $MoO_2$ (SALOPH) in Example 1(a) and 1(b) for instance, refers to a chelate complex of molybdenum oxide with phenylene bis(salicylimine).

Moreover, the complexes $MoO_2$(SAN)(acac) (Examples 2(a) and 5(b)) and $MoO_2$(SAP) (Examples 4(a) and 4(b)) referred to below were prepared by the method described by J. Topich, in Inorg. Chem., 1981, 20, 3704—3707.

The other complexes were made by ligand exchange between $MoO_2(acac)_2$ and the appropriate nitrogenous ligand in a refluxing solvent such as ethanol, toluene or tetrahydrofuran.

The ligands "PIP" and "PPB" are not examples of the ligands defined herein but are included for purposes of comparison.

### Example 1(a)

An autoclave was charged with 20 ml of a 2.78 molar solution of tert-butyl hydroperoxide in toluene and 0.1 g of $MoO_2$ (SALOPH). It was pressured to 50 bar with ethylene and heated to 90°C for 2 hours with stirring. It was then cooled to 0°C and the contents discharged. Analysis by gas chromatography and titration showed that 60% of the tert-butyl hydroperoxide had been converted with 49% selectivity to ethylene oxide based on tert-butyl hydroperoxide.

### Example 1(b)

An autoclave was charged with 20 ml of a 3.44 molar solution of tert-butyl hydroperoxide in toluene, 0.1 g $MoO_2$ (SALOPH), and 0.2 ml cyclohexene oxide. It was pressured to 49 bar with ethylene and heated to 90°C for 1 hour. The conversion of tert-butyl hydroperoxide was 22% with 67% selectivity to ethylene oxide based on tert-butyl hydroperoxide.

### Example 2(a)

An autoclave was charged with 20 ml of a 2.78 molar solution of tert-butyl hydroperoxide in toluene and 0.05 g $MoO_2$ $(salicylaldoxime)_2$. It was pressured to 50 bar with ethylene and heated to 90°C for 2 hours. The conversion of tert-butyl hydroperoxide was 77% with 51% selectivity to ethylene oxide based on tert-butyl hydroperoxide.

### Example 2(b)

An autoclave was charged with 20 ml of a 2.39 molar solution of tert-butyl hydroperoxide in toluene, 0.1 g $MoO_2$ $(salicylaldoxime)_2$ and 0.2 ml cyclohexene oxide. It was pressured to 49 bar with ethylene and

heated to 90°C for 2 hours. The conversion of tert-butyl hydroperoxide was 58% with 53% selectivity to ethylene oxide based on tert-butyl hydroperoxide.

Example 3(a)

An autoclave was charged with 20 ml of a 4.30 molar solution of tert-butyl hydroperoxide in toluene and 0.05 g $MoO_2$ (SALEN). It was pressured to 55 bar with ethylene and heated to 90°C for 2 hours. The conversion of tert-butyl hydroperoxide was 4% with 12% selectivity to ethylene oxide based on tert-butyl hydroperoxide.

Example 3(b)

An autoclave was charged with 20 ml of a 3.44 molar solution of tert-butyl hydroperoxide in toluene, 0.1 g $MoO_2$ (SALEN) and 0.2 ml cyclohexene oxide. It was pressured to 47 bar with ethylene and heated to 90°C for 1 hour. The conversion of tert-butyl hydroperoxide was 29% with 55% selectivity to ethylene oxide based on tert-butyl hydroperoxide.

Example 4(a)

An autoclave was charged with 20 ml of a 3.39 molar solution of tert-butyl hydroperoxide in toluene and 0.1 g $MoO_2$ (SAP). It was pressured to 55 bar with ethylene and heated to 90°C for 2 hours. The conversion of tert-butyl hydroperoxide was 20% with 26% selectivity to ethylene oxide based on tert-butyl hydroperoxide.

Example 4(b)

An autoclave was charged with 20 ml of a 3.44 molar solution of tert-butyl hydroperoxide in toluene, 0.1 g $MoO_2$ (SAP) and 0.2 ml cyclohexene oxide. It was pressured to 50 bar and heated to 90°C for 2 hours. The conversion of tert-butyl hydroperoxide was 54% with 44% selectivity to ethylene oxide based on tert-butyl hydroperoxide.

Example 5(a)

An autoclave was charged with 20 ml of a 3.39 molar solution of tert-butyl hydroperoxide in toluene and 0.1 g $MoO_2$ (SAN) (acac). It was pressured to 55 bar and heated to 90°C for 2 hours. The conversion of tert-butyl hydroperoxide was 62% with 29% selectivity to ethylene oxide based on tert-butyl hydroperoxide.

Example 5(b)

An autoclave was charged with 20 ml of a 3.44 molar solution of tert-butyl hydroperoxide in toluene and 0.1 g $MoO_2$ (SAN) (acac) and 0.2 ml cyclohexene oxide. It was pressured to 48 bar with ethylene and heated to 90°C for 1 hour. The conversion of tert-butyl hydroperoxide was 67% with 18% selectivity to ethylene oxide based on tert-butyl hydroperoxide.

Experiment A

An autoclave was charged with 20 ml of a 3.40 molar solution of tert-butyl hydroperoxide in toluene and 0.1 g $MoO_2$ $(PIP)_2$ and 0.2 ml cyclohexene oxide. It was pressured to 50 bar with ethylene and heated to 90°C for 2 hours. The conversion of tert-butyl hydroperoxide was 78% with 6% selectivity to ethylene oxide based on tert-butyl hydroperoxide.

Experiment B

An autoclave was charged with 20 ml of a 3.49 molar solution of tert-butyl hydroperoxide in toluene and 0.1 g $MoO_2$ (PPB) and 0.2 ml cyclohexene oxide. It was pressured to 50 bar ethylene and heated to 90°C for 2 hours. The conversion of tert-butyl hydroperoxide was 45% with 7% selectivity to ethylene oxide based on tert-butyl hydroperoxide.

Experiments A and B, which do not constitute part of the invention as described, show that other bidentate nitogenous ligands, which are not members of the class described herein, produce catalysts which are much less selective.

**Claims**

1. A process for the epoxidation of ethylene in the liquid phase using a hydroperoxide as an epoxidising agent and, as catalyst, an effective amount of a chelate complex of a Group VB or VIB metal and a bidentate nitrogenous ligand characterised in that the bidentate nitrogenous ligand has the general formula:

HO—Ar—CH=NR

wherein Ar is an arylene group with the OH group and the —CH=NR group in an ortho relationship and

where R is selected from the groups consisting of OH, —Ar—OH, —Ar—COOH, and —$R_1$—N=CH—Ar—OH and $R_1$ is either an arylene group or a —$(CH_2)n$— group where n is an integer from 0 to 4.

2. A process as claimed in claim 1 characterised in that the reaction is carried out in the presence of an epoxide.

3. A process as claimed in claim 3 characterised in that the epoxide is either ethylene oxide or cyclohexene oxide.

4. A process as claimed in claims 1 or 2 characterised in that the bidentate nitrogenous ligand is salicylaldoxime.

5. A process as claimed in claims 1 or 2 characterised in that the bidentate nitrogenous ligand is N-salicylidene-2-aminophenol.

6. A process as claimed in claims 1 or 2 characterised in that the bidentate nitrogenous ligand is phenylene bis(salicylimine).

7. A process as claimed in claims 1 or 2 characterised in that the bidentate nitrogenous ligand is ethylene bis(salicylimine).

8. A process as claimed in claim 1 characterised in that the bidentate nitrogenous ligand is N-salicylidene-2-aminobenzoic acid.

9. A process as claimed in claims 1 or 2 characterised in that the Group VIB metal is molybdenum.

10. A process as claimed in claims 1 or 2 characterised in that the molar ratio of the chelate complex to hydroperoxide is between 0.005 and 0.1.

11. A process as claimed in claim 10 characterised in that the molar ratio of the chelate complex to hydroperoxide is between 0.02 and 0.05.

12. A process as claimed in claims 1 or 2 characterised in that the olefin to hydroperoxide molar ratio is between 0.5 and 100.

13. A process as claimed in claims 1 or 2 characterised in that the reaction temperature is between 50 and 150°C.

14. A process as claimed in claims 1 or 2 characterised in that the olefin used is ethylene and the hydroperoxide is tert-butyl hydroperoxide.

15. A process as claimed in claims 1 or 2 characterised in that the reaction is carried out in an inert oxidant stable solvent.

**Patentansprüche**

1. Verfahren zur Epxodierung von Ethylen in flüssiger Phase unter Verwendung eines Hydroperoxids als epoxidierendes Mittel und, als Katalysator, einer wirksamen Menge eines Chelatkomplexes eines Metalls der Gruppe VB oder VIB und eines zweizähnigen stickstoffhaltigen Liganden, dadurch gekennzeichnet, daß der zweizähnige stickstoffhaltige Ligand die allgemeine Formel:

$$HO—Ar—CH=NR$$

hat, worin Ar eine Arylengruppe mit der OH-Gruppe und der —CH=NR-Gruppe in einer ortho-Wechselbeziehung ist und, worin R ausgewählt ist aus den Gruppen, bestehend aus OH, —Ar—OH, —Ar—COOH und —$R_1$—N=CH—Ar—OH, und $R_1$ entweder eine Arylengruppe oder eine —$(CH_2)_n$-Gruppe ist, worin n eine ganze Zahl von 0 bis 4 ist.

2. Verfahren nach Anspruch 1, dadurch gekennzeichnet, daß die Umsetzung in Gegenwart eines Epoxids durchgeführt wird.

3. Verfahren nach Anspruch 3 dadurch gekennzeichnet, daß das Epoxid entweder Ethylenoxid oder Cyclohexenoxid ist.

4. Verfahren nach Anspruch 1 oder 2, dadurch gekennzeichnet, daß der zweizähnige stickstoffhaltige Ligand Salicylaldoxim ist.

5. Verfahren nach Anspruch 1 oder 2, dadurch gekennzeichnet, daß der zweizähnige stickstoffhaltige Ligand N-Salicyliden-2-aminophenol ist.

6. Verfahren nach Anspruch 1 oder 2, dadurch gekennzeichnet, daß der zweizähnige stickstoffhaltige Ligand Phenylen- bis(salicylimin) ist.

7. Verfahren nach Anspruch 1 oder 2, dadurch gekennzeichnet, daß der zweizähnige stickstoffhaltige Ligand Ethylen- bis(salicylimin) ist.

8. Verfahren nach Anspruch 1, dadurch gekennzeichnet, daß zweizähnige stickstoffhaltige Ligand N-Salicyliden-2-aminobenzoesäure ist.

9. Verfahren nach Anspruch 1 oder 2, dadurch gekennzeichnet, daß das Metall der Gruppe VIB Molybdän ist.

10. Verfahren nach Anspruch 1 oder 2, dadurch gekennzeichnet, daß das molare Verhältnis des Chelatkomplexes zu Hydroperoxid zwischen 0,005 und 0,1 beträgt.

11. Verfahren nach Anspruch 10, dadurch gekennzeichnet, daß das molare Verhältnis des Chelatkomplexes zu Hydroperoxid zwischen 0,02 und 0,05 beträgt.

12. Verfahren nach Anspruch 1 oder 2, dadurch gekennzeichnet, daß das molare Verhältnis von Olefin zu Hydroperoxid zwischen 0,5 und 100 beträgt.

13. Verfahren nach Anspruch 1 oder 2, dadurch gekennzeichnet, daß die Reaktionstemperatur zwischen 50 und 150°C beträgt.

14. Verfahren nach Anspruch 1 oder 2, dadurch gekennzeichnet, daß das verwendete Olefin Ethylen ist und das Hydroperoxid tert.-Butylhydroperoxid ist.

15. Verfahren nach Anspruch 1 oder 2, dadurch gekennzeichnet, daß die Umsetzung in einem inerten Oxidans-stabilen Lösungsmittel durchgeführt wird.

**Revendications**

1. Procédé pour l'époxydation de l'éthylène en phase liquide, en utilisant un hydroperoxyde à titre d'agent d'époxydation et, comme catalyseur, une quantité efficace d'un chélate complexe d'un métal du groupe VB ou VIB et un ligand azoté bidenté, procédé caractérisé en ce que le ligand azoté bidenté a pour formule générale:

$$HO—Ar—CH=NR$$

dans laquelle Ar représente un groupe arylène comportant le groupe OH et le groupe —CH=NR en position ortho l'un par rapport à l'autre et R est choisi parmi les groupes consistant en OH, —Ar—OH, —Ar—COOH et $—R_1—N=CH—Ar—OH$ et $R_1$ est un groupe arylène ou un groupe $—(CH_2)n—$, ou n est un nombre entier valant de 0 à 4.

2. Procédé selon la revendication 1, caractérisé en ce qu'on effectue la réaction en présence d'un époxyde.

3. Procédé selon la revendication 2, caractérisé en ce que l'époxyde est l'oxyde d'éthylène ou l'oxyde de cyclohexène.

4. Procédé selon la revendication 1 ou 2, caractérisé en ce que le ligand azoté bidenté est le salicylaldoxime.

5. Procédé selon la revendication 1 ou 2, caractérisé en ce que le ligand azoté bidenté est le N-salicylidène-2-aminophénol.

6. Procédé selon la revendication 1 ou 2, caractérisé en ce que le ligand azoté bidenté est la phénylène bis(salicylimine).

7. Procédé selon la revendication 1 ou 2, caractérisé en ce que le ligand azoté bidenté est l'éthylène bis(salicylimine).

8. Procédé selon la revendication 1, caractérisé en ce que le ligand azoté bidenté est l'acide N-salicylidène-2-aminobenzoïque.

9. Procédé selon la revendication 1 ou 2, caractérisé en ce que le métal du groupe VIB est le molybdène.

10. Procédé selon la revendication 1 ou 2, caractérisé en ce que le rapport molaire du chélate complexe à l'hydroperoxyde se situe entre 0,005 et 0,1.

11. Procédé selon la revendication 10, caractérisé en ce que le rapport molaire du chélate complexe à l'hydroperoxyde se situe entre 0,02 et 0,05.

12. Procédé selon la revendication 1 ou 2, caractérisé en ce que le rapport molaire de l'oléfine à l'hydroperoxyde est compris entre 0,5 et 100.

13. Procédé selon la revendication 1 ou 2, caractérisé en ce que la température de réaction est comprise entre 50 et 150°C.

14. Procédé selon la revendication 1 ou 2, caractérisé en ce que l'oléfine utilisée est l'éthylène, et l'hydroperoxyde est l'hydroperoxyde de tertio-butyle.

15. Procédé selon la revendication 1 ou 2, caractérisé en ce qu'on effectue la réaction dans un solvant inerte stable en présence des oxydants.